Europäisches Patentamt

European Patent Office

Office européen des brevets

(19)

(11) Numéro de publication : **0 447 299 B1**

## (12) FASCICULE DE BREVET EUROPEEN

(45) Date de publication du fascicule du brevet :
**03.08.94 Bulletin 94/31**

(51) Int. Cl.⁵ : **G01N 33/14**

(21) Numéro de dépôt : **91400622.6**

(22) Date de dépôt : **07.03.91**

(54) **Méthode de dosage du saccharate monocalcique dans un jus sucré.**

(30) Priorité : **08.03.90 FR 9002939**

(43) Date de publication de la demande :
**18.09.91 Bulletin 91/38**

(45) Mention de la délivrance du brevet :
**03.08.94 Bulletin 94/31**

(84) Etats contractants désignés :
**AT BE CH DE DK ES GB IT LI NL SE**

(56) Documents cités :
**FR-A- 1 513 459**
**MEASUREMENT TECHNIQUES, vol. 20, no. 6,**
**juin 1977, pages 906-907; D.L. NOSOVIT-**
**SKAYA et al.: "Automatic line ULS-1 for mea-**
**suring sugar-beet saccharimeters"**

(73) Titulaire : **F C B**
**38, rue de la République**
**F-93107 Montreuil Cédex (FR)**

(72) Inventeur : **Rouanne, François**
**23, rue du Molinel**
**F-59800 Lille (FR)**

(74) Mandataire : **Fontanié, Etienne**
**FIVES-CAIL BABCOCK**
**38, rue de la République**
**F-93100 Montreuil (FR)**

EP 0 447 299 B1

## Description

La présente invention a pour objet une méthode de dosage du saccharate monocalcique dans les jus sucrés utilisable, en particulier, en sucrerie dans la production des jus sucrés par le procédé de diffusion alcaline. On sait que dans ce procédé les cossettes sont soumises à un traitement préalable de calcification au moyen d'un jus contenant du saccharate monocalcique. Pour contrôler ce traitement, il est important de connaître la teneur en saccharate monocalcique du jus utilisé, au moins en début et en fin de traitement. Or, il n'existait jusqu'à présent aucune méthode de dosage de ce composé dans un jus sucré.

La présente invention a pour but de combler cette lacune.

La méthode de dosage du saccharate monocalcique objet de l'invention consiste à prélever un échantillon du jus à analyser, diluer l'échantillon avec de l'alcool pour former un précipité, séparer le précipité par filtration, dissoudre le précipité dans de l'eau désionisée, acidifier la solution obtenue par addition d'acide phosphorique et doser le saccharose dans ladite solution.

L'alcool utilisé pour diluer l'échantillon sera, de préférence de l'éthanol.

Le saccharose sera dosé par polarimétrie, au moyen d'un saccharimètre, après avoir soumis la solution à une défécation, suivant la méthode habituellement utilisée pour le dosage du saccharose dans les jus de sucrerie et décrite, par exemple, dans le brevet FR 1 513 459.

On décrit ci-après, à titre d'exemple, un mode de mise en oeuvre pratique de l'invention.

On prélève un échantillon de 10ml du jus à analyser et on le verse dans une fiole de 100 ml, puis on complète le remplissage de la fiole à 100 ml avec de l'éthanol.

Il se forme un précipité, constitué essentiellement mais pas exclusivement par du saccharate monocalcique, que l'on sépare de la phase liquide par filtration sous vide dans un filtre à membrane, par exemple un filtre MILLIPORE (marque déposée) n AT 200 - 47 - 00, qui a été pesé au préalable.

Le précipité retenu sur la membrane du filtre est lavé avec de l'alcool puis séché avec le filtre dans une étuve pendant une à deux heures. Le filtre est ensuite pesé pour déterminer le poids du précipité.

On dissout ensuite le précipité dans 50 ml d'eau désionisée, on acidifie la solution obtenue jusqu'à pH 4 par addition d'acide phosphorique 3 N et on ajoute de l'eau désionisée pour obtenir 100ml d'une solution contenant du saccharose et du phosphate de calcium.

On soumet cette solution à une défécation et on dose le saccharose qu'elle contient par polarimétrie suivant la méthode habituellement utilisée pour le dosage du saccharose dans les jus de sucrerie.

Le poids de saccharose, en grammes, contenu dans les 10ml de l'échantillon est donné par la formule :

$$Ps = \text{Lecture du saccharimètre x coefficient d'étalonnage.}$$

En France, le coefficient d'étalonnage est égal à 0,26.

Le poids de saccharate monocalcique contenu dans les 10ml de l'échantillon sera calculé par la formule :

$$Pm = Ps \times \frac{382}{342} = Ps \times 1,117$$

Le poids de chaux dans les 10ml qui a réagi avec le saccharose sera

$$Pca = 0,1047\ Pm = 0,1170\ Ps$$

A titre de vérification, on pourra doser le calcium dans la solution au moyen d'un spectrophotomètre à absorption atomique et s'assurer que le rapport molaire $\frac{saccharose}{calcium}$ est bien égal à 1. On peut également doser le calcium par alcalinité, méthode de dosage classique en sucrerie.

## Revendications

1. Méthode de dosage du saccharate monocalcique dans un jus sucré consistant à prélever un échantillon du jus à analyser, diluer l'échantillon avec de l'alcool pour former un précipité, séparer le précipité par filtration, dissoudre le précipité dans de l'eau désionisée, acidifier la solution obtenue par addition d'acide phosphorique et doser le saccharose dans ladite solution.

2. Méthode de dosage selon la revendication 1, caractérisée en ce que l'échantillon est dilué dans 9 fois son volume d'alcool.

3. Méthode de dosage selon la revendication 1 ou 2, caractérisée en ce qu'on dilue l'échantillon avec de l'éthanol.

4. Méthode de dosage selon la revendication 1, 2 ou 3, caractérisée en ce que le pH de ladite solution est de l'ordre de 4 après acidification.

5. Méthode de dosage selon la revendication 1, 2, 3 ou 4 caractérisée en ce que ladite solution est soumise à une défécation avant dosage du saccharose par polarimétrie.

6. Méthode de dosage selon la revendication 1, 2, 3 ou 4 caractérisée en ce qu'on dose le calcium dans ladite solution et on vérifie que le rapport saccharose/calcium est égal à 1.

**Patentansprüche**

1. Methode zur Bestimmung des in zuckerhaltigem Saft enthaltenen Monokalziumsaccharats, gemäß der man eine Probe des zu analysierenden Saftes entnimmt, diese Probe mit Alkohol verdünnt, um einen Niederschlag zu erhalten, den Niederschlag abfiltert, den Niederschlag in entionisiertem Wasser auflöst, die erhaltene Lösung durch Zugabe von Phosphorsäure ansäuert und die in dieser Lösung enthaltene Saccharose bestimmt.

2. Bestimmungsmethode gemäß Anspruch 1, dadurch gekennzeichnet, daß die Probe mit 9 mal ihrem Volumen Alkohol verdünnt wird.

3. Bestimmungsmethode gemäß Anspruch 1 oder 2, dadurch gekennzeichnet, daß die Probe mit Ethanol verdünnt wird.

4. Bestimmungsmethode gemäß Anspruch 1, 2 oder 3, dadurch gekennzeichnet, daß der pH besagter Lösung nach der Ansäuerung ca. 4 beträgt.

5. Bestimmungsmethode gemäß Anspruch 1, 2, 3 oder 4, dadurch gekennzeichnet, daß die Lösung vor der polarimetrischen Saccharosebestimmung geklärt wird.

6. Bestimmungsmethode gemäß Anspruch 1, 2, 3 oder 4, dadurch gekennzeichnet, daß man das in besagter Lösung enthaltene Kalzium bestimmt und überprüft, ob das Saccharose/Kalzium-Verhältnis gleich 1 ist.

**Claims**

1. Method for determining the monocalcium saccharate in a sweet juice consisting in taking a sample of the juice to be analyzed, in diluting the sample with alcohol to form a precipitate, in separating the precipitate by filtration, in dissolving the precipitate in deionized water, in acidifying the solution obtained by adding phosphoric acid, and in determining the saccharose in the said solution.

2. Determination method according to claim 1, characterized in that the sample is diluted in a quantity of alcohol equal to 9 times its volume.

3. Determination method according to claim 1 or 2, characterized in that the sample is diluted with ethanol.

4. Determination method according to claim 1, 2 or 3, characterized in that the pH of the said solution is in the order of 4 after acidification.

5. Determination method according to claim 1, 2, 3 or 4, characterized in that the said solution is subjected to a defecation process prior to determining the saccharose by polarimetry

6. Determination method according to claim 1, 2, 3 or 4, characterized in that one determines the calcium contained in the said solution, and ascertains that the saccharose/calcium relation is equal to 1.